# EUROPÄISCHE PATENTSCHRIFT

(11) **EP 2 898 860 B1**
(45) Veröffentlichungstag und Bekanntmachung des Hinweises auf die Patenterteilung: **19.09.2018**
(21) Anmeldenummer: 15152279.4
(22) Anmeldetag: 23.01.2015
(51) Int. Cl.: A61F 2/46, A61F 2/34, A61F 2/30

(54) **Hüftgelenk-Endoprothesen-System**
Hip joint endoprosthesis system
Système d'endoprothèses de hanches

(30) Priorität: 24.01.2014 DE 102014100821
(43) Veröffentlichungstag der Anmeldung: 29.07.2015
(73) Patentinhaber: AESCULAP AG, 78532 Tuttlingen (DE)
(72) Erfinder: Schneider, Jens, 78315 Radolfzell (DE); Weiß, Josef-Benedikt, 78628 Rottweil (DE)
(74) Vertreter: Hoeger, Stellrecht & Partner Patentanwälte mbB

(56) Entgegenhaltungen:
- WO-A1-2008/106598
- WO-A1-2011/161166
- DE-A1- 19 820 721
- DE-A1-102007 055 467
- US-A1- 2007 219 640

## Beschreibung

Die vorliegende Erfindung betrifft ein Hüftgelenk-Endoprothesen-System mit einem medizinischen Instrumentarium zum Implantieren einer Hüftgelenk-Endoprothese umfassend mindestens ein medizinisches Instrument zum Einsetzen eines Gelenkeinsatzes in eine Gelenkpfanne der Hüftgelenk-Endoprothese, wobei das Instrument eine Längsachse definiert und ein Halteelement zum temporären Koppeln mit dem Gelenkeinsatz und einen Zentrierkörper zum Zentrieren des Gelenkeinsatzes relativ zum Halteelement umfasst, wobei das Halteelement mindestens ein Rückhalteglied aufweist, welches eine Rückhaltefläche aufweist.

Hüftgelenk-Endoprothesen umfassen üblicherweise eine Gelenkpfanne, die im Beckenknochen des Patienten verankert wird. Die Gelenkpfanne dient der Aufnahme eines Gelenkeinsatzes, welcher zusammen mit einem Gelenkkopf, der mit einem in einen Femur einzusetzenden Prothesenschaft verbunden ist, eine Gleitpaarung ausbildet. Die Gelenkpfanne ist vorzugsweise aus einem körperverträglichen Metall hergestellt, der Gelenkeinsatz kann wahlweise aus einem Kunststoff, beispielsweise Polyethylen mit einem ultrahohen Molekulargewicht (UHMWPE), oder aus einer Keramik hergestellt sein.

Der Gelenkeinsatz wird erst dann in die Gelenkpfanne eingesetzt, wenn diese im Beckenknochen in gewünschter Weise verankert ist. Zum Einsetzen des Gelenkeinsatzes sind medizinische Instrumente der eingangs beschriebenen Art bekannt, mit denen der Gelenkeinsatz gehalten und an die Gelenkpfanne herangeführt werden kann. Allerdings ist die Handhabung des Gelenkeinsatzes beim Einsetzen desselben in die Gelenkpfanne nicht immer besonders einfach, insbesondere das Entfernen des Instruments nach dem beispielsweise über eine selbsthemmende Klemmung mit der Gelenkpfanne gekoppelten Gelenkeinsatz macht häufig Schwierigkeiten. Weitere Probleme bei der Handhabung des Gelenkeinsatzes und der Gelenkpfanne sind zudem, insbesondere bei aus einer Keramik hergestellten Gelenkeinsätzen, ein positionsrichtiges Einsetzen sowie ein sicheres Führen derselben bis zur Endlage. Ferner wird durch bislang verfügbare Instrumentarien keine Rückmeldung ermöglicht, ob die richtige Endposition erreicht ist. Daher muss stets eine zeitaufwendige Nachkontrolle vorgenommen werden.

Aus der WO 2011/161166 A1 ist ein Einsetzinstrument zum Einsetzen von Pfanneneinsätzen in Hüftpfannen für die Hüftendoprothetik bekannt. In der DE 198 20 721 A1 ist ein Instrument zur Handhabung von Komponenten von Gelenkprothesen beschrieben. Die DE 10 2007 055 467 A1 offenbart ein chirurgisches System und Verfahren zum Verbinden eines Instruments mit einem Implantatteil. Aus der WO 2008/106598 A1 ist ein Einsetzführung für Einsätze von Hüftgelenkpfannen bekannt. In der US 2007/0219640 A1 ist eine Prothese mit Keramik-Keramik Gleitpaarung beschrieben.

Es ist daher eine Aufgabe der vorliegenden Erfindung, ein Hüftgelenk-Endoprothesen-System der eingangs beschriebenen Art so zu verbessern, dass insbesondere eine Handhabung des Gelenkeinsatzes beim Einsetzen in die Gelenkpfanne vereinfacht wird.

Diese Aufgabe wird bei einem Hüftgelenk-Endoprothesen-System der eingangs beschriebenen Art erfindungsgemäß dadurch gelöst, dass die Rückhaltefläche bezogen auf die Längsachse geneigt ist, dass die Rückhaltefläche in Form einer in Richtung auf die Längsachse hin weisenden Ringfläche ausgebildet ist und dass die Rückhaltefläche in Form einer sich in distaler Richtung verjüngenden Innenkonusfläche ausgebildet ist.

Ein Hüftgelenk-Endoprothesen-System auf diese Weise weiterzubilden hat insbesondere den Vorteil, dass der Gelenkeinsatz mit dem Zentrierkörper einfach und sicher zentriert mit dem Instrument erfasst und an diesem gehalten werden kann. So wird es einem Operateur erleichtert, den Gelenkeinsatz in definierter und gewünschter Weise, ohne zu verkanten in die Gelenkpfanne einzusetzen. Insbesondere kann nur ein einziges Halteelement vorgesehen sein, welches eine kraft- und/oder formschlüssige temporäre Kopplung mit dem Gelenkeinsatz gestattet. Im Vergleich mit Instrumenten, die drei oder mehr einzelne Halteelemente aufweisen, um den Gelenkeinsatz zu halten, kann so eine deutlich verbesserte Haltefunktion und Zentrierfunktion mit dem Instrument erreicht werden. Das mindestens eine medizinische Instrument zum Einsetzen eines Gelenkeinsatzes in eine Gelenkpfanne der Hüftgelenk-Endoprothese kann insbesondere auch mit einem bereits mit einer Gelenkpfanne gekoppelten Gelenkeinsatz in Eingriff gebracht werden, um die Einheit handzuhaben, beispielsweise um die aus Gelenkpfanne und Gelenkeinsatz bestehende Einheit in das Becken eines Patienten zu implantieren, insbesondere einzuschlagen. Es bildet dann ein medizinisches Instrument zum Einsetzen einer aus einer Gelenkpfanne und einem Gelenkeinsatz bestehenden Einheit in das Becken eines Patienten. Außerdem ist es mit dem mindestens einen medizinischen Instrument zum Einsetzen eines Gelenkeinsatzes in eine Gelenkpfanne der Hüftgelenk-Endoprothese insbesondere auch möglich, eine temporäre Kopplung, beispielsweise kraft- und/oder formschlüssig, mit einer Gelenkpfanne herzustellen, um diese in das Becken eines Patienten zu implantieren, insbesondere einzuschlagen. Es bildet dann ein medizinisches Instrument zum Einsetzen einer Gelenkpfanne in das Becken eines Patienten, welches ein Halteelement zum temporären Koppeln mit der Gelenkpfanne und einen Zentrierkörper zum Zentrieren der Gelenkpfanne relativ zum Halteelement umfasst. Die Gelenkpfanne kann insbesondere bereits eine Gleitfläche für einen Gelenkkopf umfassen, so dass kein zusätzlicher Gelenkeinsatz benötigt wird, oder eine Aufnahme für einen Gelenkeinsatz, der in der beschriebenen Weise mit dem Instrument in die Gelenkpfanne eingesetzt werden kann. Günstig ist es, dass das Halteelement mindestens ein Rückhalteglied aufweist, welches eine Rückhaltefläche aufweist, die bezogen auf die Längsachse geneigt ist. Vorzugsweise ist die Rückhaltefläche derart geneigt, dass sich eine in distaler Richtung verjüngende, insbesondere konisch verjüngende, Innenfläche durch die Rückhaltefläche definiert wird. Insbesondere kann ein Neigungswinkel der Rückhaltefläche an einen Neigungswinkel einer Außenkontur des Gelenkeinsatzes angepasst sein. Um möglichst gut Klemmkräfte zum Halten vom Instrument auf den Gelenkeinsatz übertragen zu können, ist es günstig, wenn ein Neigungswinkel, welcher zwischen einer zur Längsachse des Instruments senkrecht verlaufenden Bezugsebene und der Rückhaltefläche etwas kleiner ist als ein Neigungswinkel zwischen der konischen Außenfläche des Gelenkeinsatzes und dessen Symmetrieachse. Auf besonders sichere Weise lässt sich der Gelenkeinsatz mit dem Instrument fassen und halten dadurch, dass die Rückhaltefläche in Form einer in Richtung auf die Längsachse hin weisenden Ringfläche ausgebildet ist. So kann das Halteelement insgesamt in Form eines geschlossenen Rings ausgebildet werden, welcher insbesondere eine in distaler Richtung weisende, vom Gelenkeinsatzanschlag umfasste Ringfläche und eine hierzu geneigte, von der Rückhaltefläche umfasste Ringfläche aufweist. Das Rückhalteglied ist vorzugsweise im Bereich eines äußeren Rands des Halteelements angeordnet beziehungsweise ausgebildet. So kann insgesamt ein einziges Halteelement ausgebildet werden zum Halten des Gelenkeinsatzes. Vorteilhaft ist es, dass die Rückhaltefläche in Form einer sich in distaler Richtung verjüngenden Innenkonusfläche ausgebildet ist. Insbesondere kann sie so korrespondierend zur Außenkonusfläche des Gelenkeinsatzes geformt sein, um beispielsweise auch eine flächige Anlage des Rückhalteglieds mit der Rückhaltefläche an der Außenfläche des Gelenkeinsatzes zu ermöglichen.

Vorteilhaft ist es, wenn das mindestens eine medizinische Instrument eine Längsachse definiert und wenn das Halteelement rotationssymmetrisch oder im Wesentlichen rotationssymmetrisch bezogen auf die Längsachse ausgebildet ist. Zum einen lässt sich ein solches Instrument auf einfache Weise herstellen, zum anderen spielt auch eine Orientierung in Umfangsrichtung bezogen auf die Längsachse beim Koppeln des Instruments mit dem Gelenkeinsatz keine Rolle.

Um den Gelenkeinsatz in definierter und sicherer Weise mit dem Instrument halten zu können, ist es günstig, wenn das Halteelement mindestens einen in distaler Richtung weisenden Gelenkeinsatzanschlag zum Anlegen an den Gelenkeinsatz umfasst. Mit dem Gelenkeinsatzanschlag ist es möglich, den Gelenkeinsatz in distaler Richtung zu bewegen, da er sich am Gelenkeinsatzanschlag abstützen kann.

Vorzugsweise umfasst der mindestens eine Gelenkeinsatzanschlag eine in distaler Richtung weisende Ringfläche. Insbesondere kann es sich dabei um eine in sich geschlossene Kreisringfläche handeln. Beispielsweise kann so ein einziges Halteelement ausgebildet werden zum Halten des Gelenkeinsatzes.

Ferner ist es vorteilhaft, wenn das Halteelement mindestens eine Hinterschneidung bezogen auf eine Längsachse des Instruments aufweist. So kann insbesondere eine formschlüssige Kopplung des Instruments mit dem Gelenkeinsatz auf einfache Weise hergestellt werden. Beispielsweise wird dies dadurch erleichtert, dass der Gelenkeinsatz üblicherweise ausgehend von seinem proximalen Ende eine sich konisch verjüngende Außenkontur aufweist. Dies ermöglicht es, dass das proximale Ende des Gelenkeinsatzes in die Hinterschneidung eintauchen und darin formschlüssig und/oder kraftschlüssig gehalten werden kann.

Vorteilhafterweise begrenzen der Gelenkeinsatzanschlag und die Rückhaltefläche die Hinterschneidung. So kann mit möglichst geringer Zahl an Elementen am Instrument eine Kopplungseinrichtung beziehungsweise ein Kopplungselement ausgebildet werden, um den Gelenkeinsatz, insbesondere dessen proximales Ende, zu fassen und temporär zu halten.

Günstig ist es, wenn ein Neigungswinkel zwischen der Rückhaltefläche und der Längsachse in einem Bereich von etwa 70° bis etwa 89° liegt. Insbesondere ist es vorteilhaft, wenn der Neigungswinkel in einem Bereich von etwa 80° bis etwa 88° liegt. So können insbesondere auf einfache Weise Klemmkräfte ausgeübt werden, um den Gelenkeinsatz klemmend mit dem Halteelement zu halten.

Gemäß einer weiteren bevorzugten Ausführungsform der Erfindung kann vorgesehen sein, dass das Halteelement und/oder das Rückhalteglied mindestens abschnittsweise verformbar ausgebildet sind. Dies ermöglicht es, dass insbesondere das Rückhalteglied, wenn es in einer Grundstellung einen freien Durchmesser definiert, der kleiner ist als ein maximaler Außendurchmesser des Gelenkeinsatzes, so verformt werden kann, dass das proximale Ende des Gelenkeinsatzes mit dem Halteelement koppelbar ist, beispielsweise durch Einführen des proximalen Endes des Gelenkeinsatzes in die durch den Gelenkeinsatzanschlag und die Rückhaltefläche begrenzte Hinterschneidung.

Auf besonders einfache Weise herstellen lässt sich das Halteelement, wenn es einstückig ausgebildet ist. Beispielsweise kann es so aus einem Kunststoff durch Spritzgießen hergestellt werden.

Vorteilhafterweise ist das Halteelement aus einem Kunststoff hergestellt. Insbesondere kann aus einem sterilisierbaren, beispielsweise dampfsterilisierbaren, Kunststoff oder aus einem thermoplastischen Kunststoff hergestellt sein. Denkbar ist es auch, das Halteelement aus Silikon herzustellen. Insbesondere hat Kunststoff als Material zur Ausbildung des Halteelements den Vorteil, dass es weicher ist als der Gelenkeinsatz, so dass dieser beim Fassen und Implantieren nicht durch das Halteelement beschädigt werden kann.

Ferner ist es vorteilhaft, wenn die Rückhaltefläche von einer Außenfläche des Zentrierkörpers beabstandet ist. So ist es möglich, ein proximales Ende des Gelenkeinsatzes, welcher insbesondere schalenförmig ausgebildet sein kann, zwischen die Rückhaltefläche und dem Zentrierkörper einzuführen. Eine Zentrierung des Gelenkeinsatzes am Instrument erfolgt dann insbesondere durch die besondere Formgebung des Zentrierkörpers.

Günstig ist es, wenn ein Abstand zwischen der Rückhaltefläche und der Außenfläche des Zentrierkörpers einer Dicke des Gelenkeinsatzes im Bereich von dessen proximalem Ende entspricht oder im Wesentlichen entspricht. So ist es möglich, das proximale Ende des Gelenkeinsatzes zwischen die Rückhaltefläche und die Außenfläche des Zentrierkörpers einzuführen.

Ferner ist es vorteilhaft, wenn das Rückhalteglied von einer Grundstellung, in welcher es eine in sich in distaler Richtung verjüngende Kegelmantelwand definiert, in eine Lösestellung aufspreizbar ist, in welcher das Rückhalteglied eine zur Längsachse im Wesentlichen konzentrische Zylindermantelwand definiert. So kann durch die Verformbarkeit, insbesondere Aufspreizbarkeit, des Rückhalteglieds eine durch einen distalen Rand desselben definierte Einführöffnung vergrößert werden, um den Gelenkeinsatz, der vorzugsweise einen größeren maximalen Außendurchmesser aufweist als die vom Rückhalteglied in der Grundstellung definierte Einführöffnung, durch die Einführöffnung in die Hinterschneidung des Halteelements einzuführen.

Vorzugsweise umfasst das Instrument einen Haltekörper, an welchem das Halteelement angeordnet ist und in distaler Richtung weisend vorsteht. Beispielsweise kann der Haltekörper mit einem Schaft des Instruments verbunden sein. Zudem dient er insbesondere dazu, das Halteelement in gewünschter Weise zu halten, um dieses mit einem Gelenkeinsatz zu koppeln.

Günstig ist es, wenn das Halteelement über mindestens zwei sich im Wesentlichen in radialer Richtung erstreckende Verbindungsglieder mit dem Haltekörper verbunden ist. Denkbar ist es insbesondere, zwei, drei, vier, fünf oder mehr Verbindungsglieder vorzusehen. Beispielsweise können diese stegförmig oder speichenförmig ausgebildet sein. Derartige Verbindungsglieder vorzusehen hat insbesondere den Vorteil, dass zwischen den Verbindungsgliedern Durchbrechungen definiert werden können, durch die ein Operateur freie Sicht oder im Wesentlichen freie Sicht beispielsweise auf den Gelenkeinsatz oder den Zentrierkörper hat.

Die Stabilität des Instruments lässt sich insbesondere dadurch verbessern, dass der Haltekörper und die Verbindungsglieder einstückig ausgebildet sind. Zudem lässt sich so das Instrument mit einer minimalen Zahl an Teilen ausbilden.

Ferner ist es vorteilhaft, wenn das Halteelement und die Verbindungsglieder einstückig ausgebildet sind. Auch diese Ausgestaltung verbessert die Stabilität des Instruments und reduziert zudem die Zahl der erforderlichen Teile zur Ausbildung des Instruments.

Günstig ist es zudem, wenn der Haltekörper einstückig ausgebildet ist. Auch dies verbessert die Stabilität des Instruments und reduziert die zu dessen Ausbildung erforderliche Zahl an Teilen.

Gemäß einer weiteren bevorzugten Ausführungsform der Erfindung kann vorgesehen sein, dass der Zentrierkörper am Haltekörper angeordnet oder ausgebildet ist und in distaler Richtung weisend absteht. Der Zentrierkörper und der Haltekörper können als separate Bauteile ausgebildet sein, beispielsweise sogar aus zwei unterschiedlichen Werkstoffen.

Ferner ist es günstig, wenn der Zentrierkörper über den mindestens einen Gelenkeinsatzanschlag in distaler Richtung vorstehend ausgebildet ist. Außerdem kann der Zentrierkörper auch in distaler Richtung über das Rückhalteglied vorstehen. So ist es möglich, dass beim Koppeln des Instruments mit dem Gelenkeinsatz zunächst der Zentrierkörper mit dem Gelenkeinsatz in Kontakt tritt und erst dann eine Kopplung des Gelenkeinsatzes mit dem Halteelement erfolgt beziehungsweise erfolgen kann.

Eine besonders einfache Zentrierung des Instruments beim Koppeln mit dem Gelenkeinsatz lässt sich dadurch erreichen, dass der Zentrierkörper kugelig oder im Wesentlichen kugelig ausgebildet ist oder mindestens eine Ringfläche aufweist, welche einen Teil einer Kugeloberfläche bildet. So ist es praktisch gleichgültig, ob das Instrument mit seiner Längsachse zur Symmetrieachse des Gelenkeinsatzes beim Koppeln ausgerichtet ist oder nicht. Ist dies nicht der Fall, zentriert sich der Zentrierkörper zum Gelenkeinsatz selbst, so dass quasi automatisch eine Ausrichtung der Längsachse zur Symmetrieachse erfolgt.

Der Aufbau des Instruments lässt sich weiter vereinfachen, wenn der Zentrierkörper und der Haltekörper einstückig ausgebildet sind.

Ferner ist es vorteilhaft, wenn der Zentrierkörper und der Haltekörper unlösbar miteinander verbunden sind. Dies ermöglicht es, den Zentrierkörper und den Haltekörper aus zwei unterschiedlichen Materialien herzustellen, diese dann aber insbesondere so miteinander zu verbinden, dass sie nicht voneinander gelöst werden können.

Günstig ist es, wenn das Instrument eine Verbindungseinrichtung zum Verbinden des Zentrierkörpers und des Haltekörpers umfasst. Beispielsweise kann so das Instrument zum Reinigen auf einfache Weise zerlegt werden. Zudem ist es möglich, den Zentrierkörper und den Haltekörper aus unterschiedlichen Materialien auszubilden, beispielsweise aus unterschiedlichen Kunststoffen oder Metallen.

Auf einfache Weise lässt sich der Haltekörper mit dem Zentrierkörper verbinden, wenn die Verbindungseinrichtung in Form einer Rast-, Bajonett- und/oder Schraubverbindungseinrichtung ausgebildet ist. Die Verbindungseinrichtung ermöglicht so insbesondere eine kraft- und/oder formschlüssige Verbindung des Haltekörpers und des Zentrierkörpers miteinander.

Günstig ist es, wenn der Haltekörper einen über die Verbindungsglieder in distaler Richtung vorstehenden Außengewindeabschnitt aufweist und wenn der Zentrierkörper einen zum Außengewindeabschnitt korrespondierenden Innengewindeabschnitt aufweist. Diese Ausgestaltung ermöglicht es insbesondere, den Zentrierkörper auf den Haltekörper aufzuschrauben.

Das Verbinden des Zentrierkörpers mit dem Haltekörper wird besonders einfach, wenn der Zentrierkörper eine mit dem Innengewindeabschnitt versehene Ausnehmung oder Durchbrechung aufweist. Insbesondere kann die Ausnehmung in Form einer Bohrung, beispielsweise einer Sacklochbohrung, ausgebildet sein.

Um die Stabilität des Instruments weiter zu verbessern, ist es günstig, wenn der Zentrierkörper eine in proximaler Richtung weisende Stützfläche aufweist, an welcher die mindestens zwei Verbindungsglieder in der Grundstellung anliegen. Mit anderen Worten kann somit die Stützfläche zum Abstützen der Verbindungsglieder dienen, welche das Halteelement und den Haltekörper miteinander verbinden.

Die Herstellung des Instruments kann insbesondere weiter vereinfacht werden, wenn die Stützfläche eben ist. So können die Verbindungsglieder in der Grundstellung insbesondere ebenfalls als ebene Stege ausgebildet werden, die dann im Wesentlichen flächig an der Stützfläche anliegen können.

Zur Handhabung des Instruments ist es vorteilhaft, wenn es einen Schaft aufweist, an dessen distalem Ende der Haltekörper angeordnet oder ausgebildet ist. So kann ein Operateur einen proximalen Endbereich des Schafts, welcher insbesondere in Form eines Griffs ausgebildet sein oder einen solchen tragen kann, fassen und damit einen mit dem distalen Ende des Instruments gekoppelten Gelenkeinsatz handhaben.

Ferner ist es günstig, wenn das Instrument eine in proximaler Richtung weisende Schlagfläche aufweist, welche an einem proximalen Ende des Schafts angeordnet oder ausgebildet ist. Diese Ausgestaltung ermöglicht es insbesondere, mit einem Schlagwerkzeug einen Impuls auf das Instrument zu übertragen, um einen mit einem distalen Ende des Instruments gekoppelten Gelenkeinsatz in die dafür vorgesehene Ausnehmung der Gelenkpfanne einzuschlagen, um so eine Konus-Klemmverbindung zwischen dem Gelenkeinsatz und der Gelenkpfanne herzustellen.

Die Handhabung des Instruments wird besonders einfach, wenn der Schaft die Längsachse definiert. So kann ein Operateur direkt aus der Orientierung des Schafts auf eine Orientierung des Gelenkeinsatzes schließen. Dies ist insbesondere dann vorteilhaft, wenn das Instrument zum minimalinvasiven Implantieren einer Hüftgelenk-Endoprothese genutzt wird.

Günstigerweise umfasst das Hüftgelenk-Endoprothesen-System eine Hüftgelenk-Endoprothese umfassend mindestens eine Gelenkpfanne und mindestens einen zu dieser korrespondierend ausgebildeten Gelenkeinsatz. Insbesondere dann, wenn die Hüftgelenk-Endoprothese modular ausgebildet ist, kann das Hüftgelenk-Endoprothesen-System mehrere Gelenkpfannen und mehrere Gelenkeinsätze umfassen. Des Weiteren kann das Hüftgelenk-Endoprothesen-System ein oder mehrere Schäfte und mit diesen koppelbare Gelenkköpfe umfassen. Die Gelenkköpfe sind insbesondere korrespondierend zu den verfügbaren Gelenkeinsätzen ausgebildet.

Die nachstehende Beschreibung bevorzugter Ausführungsformen der Erfindung dient im Zusammenhang mit den Zeichnungen der näheren Erläuterung. Es zeigen:
- Figur 1:: eine schematische perspektivische Ansicht eines Teils eines Hüftgelenk-End oprothesen-Systems;
- Figur 2:: eine schematische Längsschnittansicht eines medizinischen Instruments zum Einsetzen eines Gelenkeinsatzes in eine Gelenkpfanne;
- Figur 3:: eine Ansicht ähnlich Figur 2, jedoch bei in die Gelenkpfanne eingeschobenem Gelenkeinsatz und vom Gelenkeinsatz entkoppeltem Halteelement;
- Figur 4:: eine schematische Längsschnittansicht eines Teils einer Hüftgelenk-Endoprothese.

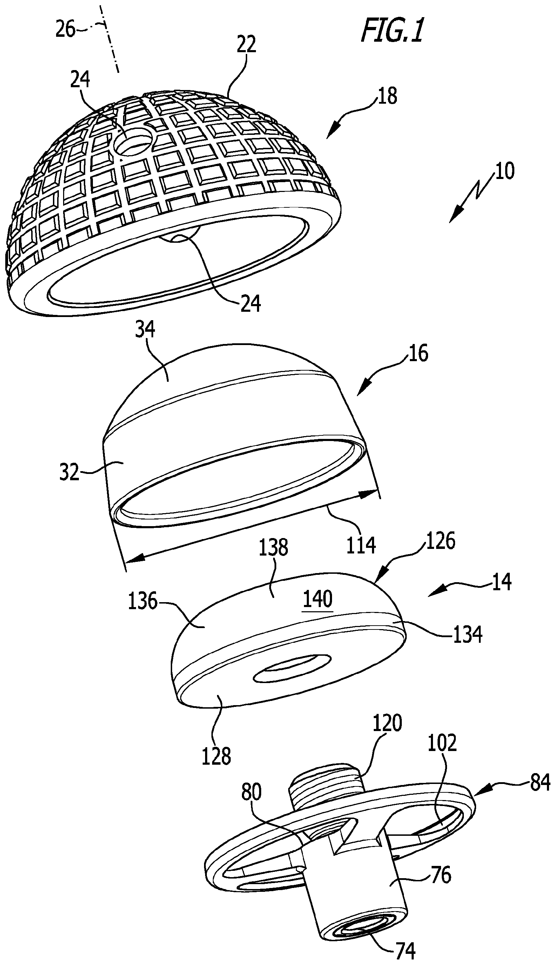

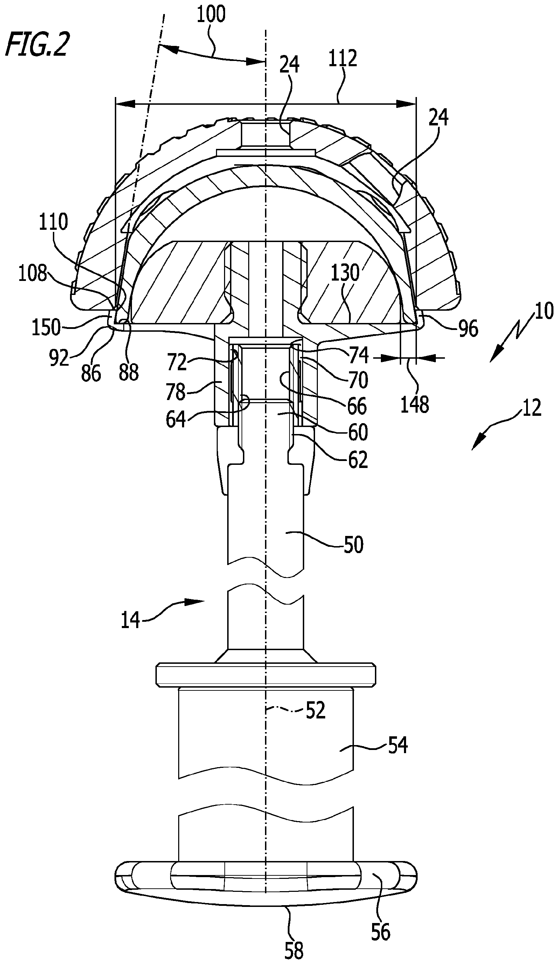

In den Figuren 1 und 2 ist beispielhaft ein insgesamt mit den Bezugszeichen 10 bezeichnetes Hüftgelenk-Endoprothesen-System dargestellt. Es umfasst insbesondere ein medizinisches Instrumentarium 12, welches insbesondere ein medizinisches Instrument 14 zum Handhaben eines Gelenkeinsatzes 16 einer Gelenkpfanne 18 einer Hüftgelenk-Endoprothese 20 umfasst.

Das Instrument 14 ist derart gestaltet, dass es zum Einsetzen des Gelenkeinsatzes 16 in die Gelenkpfanne 18 eingesetzt werden kann.

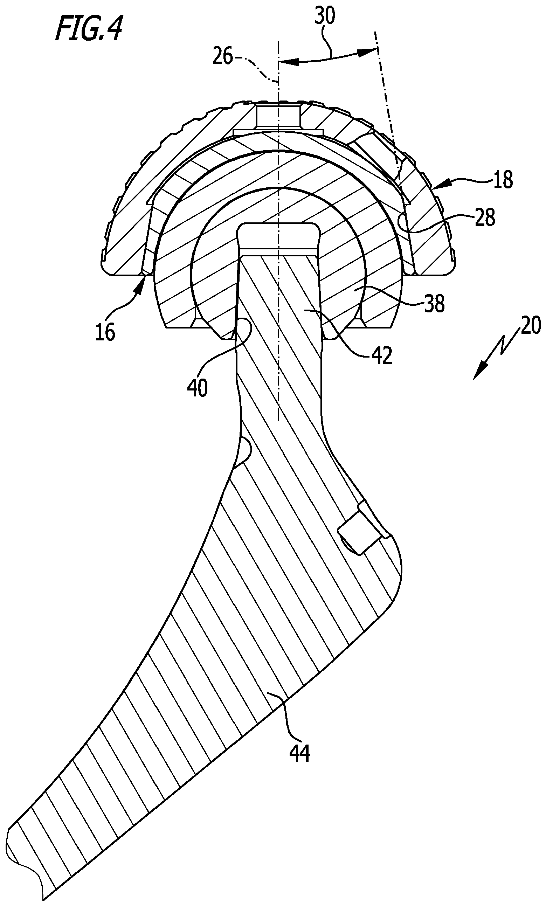

In Figur 4 ist der Aufbau der Hüftgelenk-Endoprothese 20 schematisch dargestellt. Die Gelenkpfanne 18 ist im Wesentlichen in Form einer halben Hohlkugel ausgebildet, deren Außenfläche 22 eine Struktur aufweisen kann, um das Einwachsen in den Beckenknochen eines Patienten zu verbessern. Ferner sind an der Gelenkpfanne 18 mehrere Durchbrechungen 24 angeordnet, insbesondere eine Durchbrechung 24 koaxial zu einer von der Gelenkpfanne 18 definierten Symmetrieachse 26.

Der Gelenkeinsatz 16 ist wahlweise aus Kunststoff, beispielsweise Polyethylen mit ultrahohem Molekulargewicht (UHMWPE), oder einer Keramik gefertigt. Seine Außenkontur ist an eine Innenkontur der Gelenkpfanne 18 angepasst. Diese weist eine ringförmig in sich geschlossene, sich konisch verjüngende Innenfläche 28 auf, welche mit der Symmetrieachse 26 einen Winkel 30 einschließt, der einen Wert in einem Bereich von etwa 7° bis etwa 11° aufweist.

Zur Innenfläche 28 entsprechend ist am Gelenkeinsatz 16 eine korrespondierende, sich konisch verjüngende Außenfläche 32 vorgesehen. Der verbleibende äußere Bereich 34 des Gelenkeinsatzes 16 ist im Wesentlichen in Form eines Ausschnitts einer Kugeloberfläche ausgebildet. Eine Innenfläche 36 des Gelenkeinsatzes 16 ist in Form eines Ausschnitts einer Hohlkugel geformt.

Der Gelenkeinsatz 16 dient zu Aufnahme eines im Wesentlichen kugelförmigen Gelenkkopfes 38, welcher eine Aufnahme 40 für einen Hals 42 eines Prothesenschaft 44 aufweist, der in den Markraum eines teilresezierten Femurs eingesetzt wird.

Die Hüftgelenk-Endoprothese 20 ist insgesamt modular ausgebildet, so dass entweder der Gelenkkopf 38 direkt in einen korrespondierenden Gelenkeinsatz 16 eingesetzt werden kann, oder, wie beispielhaft in Figur 4 dargestellt, eine Adapterschale 46 auf den Gelenkkopf aufgesetzt werden kann, welche wiederum eine kugelige Außenfläche 48 aufweist, die korrespondierend zur Innenfläche 36 ausgebildet ist und mit dem Gelenkeinsatz 16 eine Gleitpaarung bildet. Die Adapterschale 46 ist in nicht näher dargestellter Weise drehfest mit dem Gelenkkopf 38 koppelbar.

Das Instrument 14 umfasst einen langgestreckten, stabförmigen Schaft 50 welcher eine Längsachse 52 definiert. An ein proximales Ende des Schafts 50 schließt sich ein im Durchmesser erweiterter Griffbereich 54 an, welcher proximalseitig eine Endplatte 56 mit einer in proximaler Richtung weisenden, schwach konvexen gewölbten Schlagfläche 58 trägt.

Ein distales Ende des Schafts 50 ist in Form eines Gewindebolzens 60 mit einem Außengewinde 62 ausgebildet. Der Gewindebolzen 60 ist in eine ein zum Außengewinde 62 korrespondierendes Innengewinde 64 umfassende Aufnahme 66 eines hülsenförmigen Adapterelements 68 eingeschraubt. Das Adapterelement 68 ist mit einem Außengewinde 70 versehen, welches korrespondierend zu einem Innengewinde 72 einer sacklochförmigen Aufnahme 74 eines Haltekörpers 76 ausgebildet ist.

Der Haltekörper 76 ist in Form einer zylindrischen Hülse 78 ausgebildet, von deren distalem Ende 80 drei in radialer Richtung von der Längsachse 52 weg weisende, stegförmige Verbindungsglieder 82 abstehen. Über die Verbindungsglieder 82 ist der Haltekörper 76 mit einem konzentrisch zur Längsachse 52 ausgebildeten Halteelement 84 verbunden. In einem Längsschnitt ist das Halteelement 84 im Wesentlichen L-förmig. Es umfasst einen schmalen Kreisring 86, welcher eine in distaler Richtung weisende, in sich geschlossene Kreisringfläche 88 definiert. Der Kreisring 86 bildet mit der Ringfläche 88 einen in distaler Richtung weisenden Gelenkeinsatzanschlag 90 zum Anlegen an den Gelenkeinsatz 16 in einer nachfolgend noch näher beschriebenen Weise.

Von einem äußeren Rand 92 des Kreisrings 86 erstreckt sich in distaler Richtung weisend ein Rückhalteglied 94 in Form eines umlaufenden Rands 96, welcher eine in Richtung auf die Längsachse hin weisende Rückhaltefläche 98 aufweist. Die Rückhaltefläche 98 ist bezogen auf die Längsachse 52 geneigt. Ein Neigungswinkel 100 zwischen der Rückhaltefläche 98 und der Längsachse 52 liegt vorzugweise in einem Bereich von etwa 1° bis etwa 20°. Bei dem in den Figuren dargestellten Ausführungsbeispiel beträgt der Neigungswinkel etwa 7°.

Die Rückhaltefläche 98 ist in Form einer in Richtung auf die Längsachse 52 hin weisenden, in sich geschlossenen Ringfläche 102 ausgebildet. Aufgrund der Neigung der Rückhaltefläche 98 bezogen auf die Längsachse 52 bildet die Ringfläche 102 eine sich in distaler Richtung verjüngende Innenkonusfläche 104.

Das Halteelement 84 definiert ferner eine Hinterschneidung 106 bezogen auf die Längsachse 52 des Instruments14. Die Hinterschneidung 106 wird begrenzt in proximaler Richtung durch den Gelenkeinsatzanschlag 90 und in radialer Richtung außen durch die Rückhaltefläche 98. Eine in distaler Richtung weisende, ringförmige Randfläche 108 des Rückhalteglieds 94 begrenzt eine Einführöffnung 110, welche in einer Grundstellung des Halteelements 84, in welcher dieses nicht verformt ist, einen Innendurchmesser 112 definiert. Der Innendurchmesser 112 ist etwas kleiner als ein Außendurchmesser 114 einer in proximaler Richtung weisenden, kreisringförmigen Endfläche 116 des Gelenkeinsatzes 16.

Vom Haltekörper 76 steht in distaler Richtung weisend eine Schraubhülse 118 ab, deren Außendurchmesser etwas kleiner ist als ein Außendurchmesser des Haltekörpers 76. Die Verbindungsglieder 82 enden im Übergangsbereich zwischen dem Haltekörper 76 und der Schraubhülse 118.

Die Schraubhülse 118 ist mit einem Außengewinde 120 versehen, welches korrespondierend zu einem Innengewinde 122 einer Bohrung 124 an einem Zentrierkörper 126 ausgebildet ist. Der Zentrierkörper 126 ist bezogen auf die Längsachse 52 rotationsymmetrisch ausgebildet und weist eine in proximaler Richtung weisende, ebene Stützfläche 128 auf, an welcher in einer Grundstellung des Instruments 14, die beispielsweise in Figur 2 schematisch dargestellt ist, die Verbindungsglieder 82 flächig mit ihren in distaler Richtung weisenden Seitenflächen 130 anliegen.

Distalseitig weist der Zentrierkörper 126 eine ebenfalls ebene Endfläche 132 auf, die eine senkrecht zur Längsachse 52 verlaufende Ebene definiert. Die Endfläche 132 und die Stützfläche 128 verlaufen parallel zueinander.

An die Stützfläche 128 schließt sich ein schmaler, in radialer Richtung von der Längsachse 52 weg weisender zylindrischer Außenflächenbereich 134 an. Ein zwischen dem Außenflächenbereich 134 und der Endfläche 132 verbleibender Flächenbereich 136 ist vom Zentrierkörper 126 weg weisend konvex gekrümmt. Insbesondere kann er einen Ausschnitt einer Kugeloberfläche definieren. Der Flächenbereich 136 bildet eine Zentrierfläche 138, die in Form einer Ringfläche 140 ausgebildet ist, welche wie erwähnt, optional einen Teil einer Kugeloberfläche bilden kann.

Wie beschrieben können der Zentrierkörper 126 und der Haltekörper 76 miteinander verbunden werden. Hierfür dient eine Verbindungseinrichtung 142, die wahlweise in Form einer Rast-, Bajonett-, und/oder Schraubverbindungseinrichtung ausgebildet sein kann. Bei dem in den Figuren dargestellten Ausführungsbeispiel ist die Verbindungseinrichtung 142 in Form einer Schraubverbindungseinrichtung 144 ausgebildet. Die Schraubverbindungseinrichtung 144 umfasst das Außengewinde 120 und das Innengewinde 122.

Die Funktionsweise des Hüftgelenk-Endoprothesen-Systems 10, insbesondere des Instruments 14, wird nachfolgend in Verbindung mit den Figuren kurz erläutert.

Ist die Gelenkpfanne 18 bereits in den Beckenknochen des Patienten eingeschlagen, so kann in einem nächsten Operationsschritt der Gelenkeinsatz 16, der auch als Inlay bezeichnet wird, in die Gelenkpfanne 18 eingesetzt werden. Hierzu wird das Instrument 14 zunächst mit dem Halteelement 84 mit dem Gelenkeinsatz 16 gekoppelt. Das Halteelement 84, die Verbindungsglieder 82 und der Haltekörper 76 mit der Schraubhülse 118 sind vorzugsweise einstückig ausgebildet, und zwar aus einem Kunststoffmaterial. Beispielsweise kann diese Einheit durch Spritzgießen hergestellt sein. Günstigerweise wird dabei ein Kunststoff gewählt, der eine mindestens abschnittsweise Verformbarkeit des Halteelements 84 und/oder des Halteglieds 94 und/oder der Verbindungsglieder 82 ermöglicht.

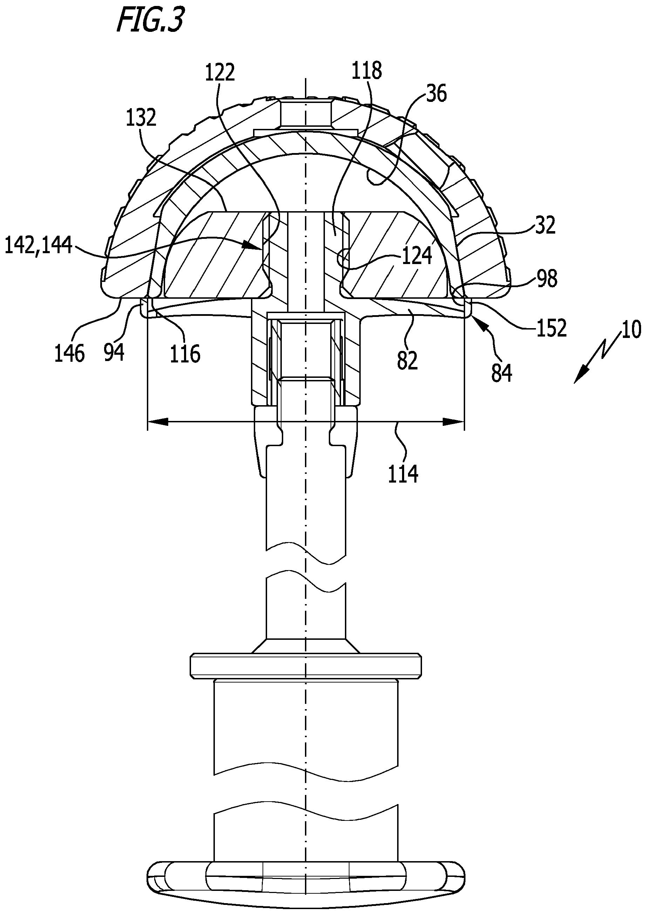

Zum Koppeln des Instruments 14 mit dem Gelenkeinsatz 16 wird das Rückhalteglied 94 von der Längsachse 52 weg derart aufgeweitet, dass sich die Einführöffnung 110 so weit aufweitet, bis der von ihr definierte Innendurchmesser dem Außendurchmesser 114 entspricht.
Das Rückhalteglied 94 geht dabei von einer schematisch in Figur 2 dargestellten Grundstellung, in welcher es eine sich in in distaler Richtung verjüngende Kegelmantelwand 150 definiert, in eine in Figur 3 schematisch dargestellte Lösestellung über, in welcher das Rückhalteglied 94 eine zur Längsachse 52 im Wesentlichen konzentrische Zylindermantelwand 152 definiert.

Das proximale Ende des Gelenkeinsatzes 16 kann nun mit der Endfläche 116 voran in die Hinterschneidung 106 eingeführt werden, und zwar bis insbesondere die Endfläche 116 an der Ringfläche 88 des Gelenkeinsatzanschlages 90 anliegt. Der Gelenkeinsatz 16 ist nun kraft- und/oder formschlüssig am Instrument 14 in der in Figur 2 dargestellten Weise gehalten. Die Endfläche 116 beziehungsweise das proximale Ende des Gelenkeinsatzes 16 taucht zwischen den Zentrierkörper 126 und das Rückhalteglied 94 ein. Dies ist möglich, da ein Abstand 148 zwischen der Rückhaltefläche 98 und der Außenfläche 134 mindestens etwa einer Dicke des Gelenkeinsatzes 16 im Bereich von dessen proximalem Ende entspricht. Das Halteelement 84 umgibt mit dem Rückhalteglied 94 den gesamten proximalen Rand des Gelenkeinsatzes 16.

Der Gelenkeinsatz 16 kann nun in gewünschter Weise vom Operateur manipuliert werden. Das Einführen des Zentrierkörpers 126 in die vom Gelenkeinsatz 16 definierte Ausnehmung ermöglicht eine optimale Positionierung des Gelenkeinsatzes 16 am Instrument 14, insbesondere eine praktisch automatische Ausrichtung der Längsachse 52 zur Symmetrieachse 26.

In einem nächsten Schritt kann der Gelenkeinsatz 16 mit dem Instrument 14 in die Gelenkpfanne 18 eingeführt werden. Zur Herstellung einer selbsthemmenden Klemmverbindung zwischen dem Gelenkeinsatz 16 und der Gelenkpfanne 18 wird mit einem in den Figuren nicht dargestellten Schlagwerkzeug auf die Schlagfläche 58 ein Schlag ausgeübt. Der Schlagimpuls wird über den Schaft 50 auf den Haltekörper 76 und von diesem über den Zentrierkörper 126 auf den Gelenkeinsatz 16 übertragen. Der Gelenkeinsatz 16 ist nun klemmend in der Gelenkpfanne 18 gehalten.

Beim Eintauchen des Gelenkeinsatzes 16 in die Gelenkpfanne 18 kommt die Randfläche 108 mit einer in proximaler Richtung weisenden, kreisringförmigen Endfläche 146 der Gelenkpfanne 18 in Kontakt. Die Endfläche 146 bildet somit einen Anschlag für das Rückhalteglied 94. Wird das Instrument 14 weiter in distaler Richtung bewegt, so werden automatisch das Rückhalteglied 94 und/oder die Verbindungsglieder 82 etwas aus ihrer Grundstellung in proximaler Richtung verformt, wie dies schematisch in Figur 3 dargestellt ist. Damit gibt das Halteelement 84 den Gelenkeinsatz 16 automatisch dann frei, wenn dieser in gewünschter Weise in die Gelenkpfanne 18 eingesetzt wird.

Das Instrument 14 kann nun in proximaler Richtung vom Gelenkeinsatz 16 abgezogen werden. Hierfür bedarf es keiner weiteren Instrumente oder einer Manipulation am Instrument 14 oder am Gelenkeinsatz 16 durch einen Operateur von Hand.

Die modulare Ausgestaltung des Instruments 14 ermöglicht es ferner, unterschiedliche Werkstoffe zu dessen Ausbildung zu nutzen. Insbesondere können alle Teile, die Schlagimpulse übertragen, vorzugsweise aus einem Metall, insbesondere aus einem sterilisierbaren Metall, hergestellt werden. Denkbar ist es aber auch, insbesondere um eine Beschädigung des Gelenkeinsatzes 16 zu vermeiden, nicht nur den Haltekörper 76, die Verbindungsglieder 82 und das Halteelement 84 aus einem Kunststoff herzustellen, sondern auch den Zentrierkörper 126.

### Bezugszeichenliste

- 10: Hüftgelenk-Endoprothesen-System
- 12: Instrumentarium
- 14: Instrument
- 16: Gelenkeinsatz
- 18: Gelenkpfanne
- 20: Hüftgelenk-Endoprothese
- 22: Außenfläche
- 24: Durchbrechung
- 26: Symmetrieachse
- 28: Innenfläche
- 30: Winkel
- 32: Außenfläche
- 34: Bereich
- 36: Innenfläche
- 38: Gelenkkopf
- 40: Aufnahme
- 42: Hals
- 44: Prothesenschaft
- 46: Adapterschale
- 48: Außenfläche
- 50: Schaft
- 52: Längsachse
- 54: Griffbereich
- 56: Endplatte
- 58: Schlagfläche
- 60: Gewindebolzen
- 62: Außengewinde
- 64: Innengewinde
- 66: Aufnahme
- 68: Adapterelement
- 70: Außengewinde
- 72: Innengewinde
- 74: Aufnahme
- 76: Haltekörper
- 78: Hülse
- 80: Ende
- 82: Verbindungsglied
- 84: Halteelement
- 86: Kreisring
- 88: Ringfläche
- 90: Gelenkeinsatzanschlag
- 92: Rand
- 94: Rückhalteglied
- 96: Rand
- 98: Rückhaltefläche
- 100: Neigungswinkel
- 102: Ringfläche
- 104: Innenkonusfläche
- 106: Hinterschneidung
- 108: Randfläche
- 110: Einführöffnung
- 112: Innendurchmesser
- 114: Außendurchmesser
- 116: Endfläche
- 118: Schraubhülse
- 120: Außengewinde
- 122: Innengewinde
- 124: Bohrung
- 126: Zentrierkörper
- 128: Stützfläche
- 130: Seitenfläche
- 132: Endfläche
- 134: Außenflächenbereich
- 136: Flächenbereich
- 138: Zentrierfläche
- 140: Ringfläche
- 142: Verbindungseinrichtung
- 144: Schraubverbindungseinrichtung
- 146: Endfläche
- 148: Abstand
- 150: Kegelmantelwand
- 152: Zylindermantelwand

## Patentansprüche

1. Hüftgelenk-Endoprothesen-System (10) mit einem medizinischen Instrumentarium (12) zum Implantieren einer Hüftgelenk-Endoprothese (20) umfassend mindestens ein medizinisches Instrument (14) zum Einsetzen eines Gelenkeinsatzes (16) in eine Gelenkpfanne (18) der Hüftgelenk-Endoprothese (20), wobei das Instrument (14) eine Längsachse (52) definiert und ein Halteelement (84) zum temporären Koppeln mit dem Gelenkeinsatz (16) und einen Zentrierkörper (126) zum Zentrieren des Gelenkeinsatzes (16) relativ zum Halteelement (84) umfasst, wobei das Halteelement (84) mindestens ein Rückhalteglied (94) aufweist, welches eine Rückhaltefläche (98) aufweist, **dadurch gekennzeichnet, dass** die Rückhaltefläche (98) bezogen auf die Längsachse (52) geneigt ist, dass die Rückhaltefläche (98) in Form einer in Richtung auf die Längsachse (52) hin weisenden Ringfläche (102) ausgebildet ist und dass die Rückhaltefläche (98) in Form einer sich in distaler Richtung verjüngenden Innenkonusfläche (104) ausgebildet ist.

2. Hüftgelenk-Endoprothesen-System nach Anspruch 1, **dadurch gekennzeichnet, dass** das Halteelement (84) rotationssymmetrisch oder im Wesentlichen rotationssymmetrisch bezogen auf die Längsachse (52) ausgebildet ist.

3. Hüftgelenk-Endoprothesen-System nach Anspruch 1 oder 2, **dadurch gekennzeichnet, dass** das Halteelement (84) mindestens einen in distaler Richtung weisenden Gelenkeinsatzanschlag (90) zum Anlegen an den Gelenkeinsatz (16) umfasst,
wobei insbesondere
a) der mindestens eine Gelenkeinsatzanschlag (90) eine in distaler Richtung weisende Ringfläche (88) umfasst
und/oder
b) der Zentrierkörper (126) über den mindestens einen Gelenkeinsatzanschlag (90) in distaler Richtung vorstehend ausgebildet ist.

4. Hüftgelenk-Endoprothesen-System nach einem der voranstehenden Ansprüche, **dadurch gekennzeichnet, dass** das Halteelement mindestens eine Hinterschneidung (106) bezogen auf eine Längsachse (52) des Instruments (14) aufweist.

5. Hüftgelenk-Endoprothesen-System nach einem der voranstehenden Ansprüche, **dadurch gekennzeichnet, dass**
a) das Halteelement (84) mindestens abschnittsweise verformbar ausgebildet ist
und/oder
b) das Rückhalteglied (94) mindestens abschnittsweise verformbar ausgebildet ist
und/oder
c) das Halteelement (84) einstückig ausgebildet ist.

6. Hüftgelenk-Endoprothesen-System nach einem der voranstehenden Ansprüche, **dadurch gekennzeichnet, dass**
a) die Rückhaltefläche (98) von einer Außenfläche (134) des Zentrierkörpers (126) beabstandet ist
und/oder
b) das Rückhalteglied (94) von einer Grundstellung, in welcher es eine sich in distaler Richtung verjüngende Kegelmantelwand (150) definiert, in eine Lösestellung aufspreizbar ist, in welcher das Rückhalteglied (94) eine zur Längsachse (52) im Wesentlichen konzentrische Zylindermantelwand (152) definiert.

7. Hüftgelenk-Endoprothesen-System nach einem der voranstehenden Ansprüche, **dadurch gekennzeichnet, dass** der Zentrierkörper (126) kugelig oder im Wesentlichen kugelig ausgebildet ist oder mindestens eine Ringfläche (140) aufweist, welche einen Teil einer Kugeloberfläche bildet.

8. Hüftgelenk-Endoprothesen-System nach einem der voranstehenden Ansprüche, **dadurch gekennzeichnet, dass** das Instrument (14) einen Haltekörper (76) umfasst, an welchem das Halteelement (84) angeordnet ist und in distaler Richtung weisend vorsteht.

9. Hüftgelenk-Endoprothesen-System nach Anspruch 8, **dadurch gekennzeichnet, dass**
a) das Halteelement (84) über mindestens zwei sich im Wesentlichen in radialer Richtung erstreckende Verbindungsglieder (82) mit dem Haltekörper (76) verbunden ist
und/oder
b) der Haltekörper (76) einstückig ausgebildet ist.

10. Hüftgelenk-Endoprothesen-System nach Anspruch 8, **dadurch gekennzeichnet, dass** der Zentrierkörper (126) am Haltekörper (76) angeordnet oder ausgebildet ist und in distaler Richtung weisend absteht.

11. Hüftgelenk-Endoprothesen-System nach Anspruch 8 oder 9, **dadurch gekennzeichnet, dass**
a) der Zentrierkörper (126) und der Haltekörper (76) unlösbar miteinander verbunden sind
und/oder
b) das Instrument (14) eine Verbindungseinrichtung zum Verbinden des Zentrierkörpers (126) und des Haltekörpers (76) umfasst.

12. Hüftgelenk-Endoprothesen-System nach einem der voranstehenden Ansprüche, **dadurch gekennzeichnet, dass** der Zentrierkörper (128) eine in proximaler Richtung weisende Stützfläche (128) aufweist, an welcher die mindestens zwei Verbindungsglieder (82) in der Grundstellung anliegen.

13. Hüftgelenk-Endoprothesen-System nach einem der Ansprüche 7 bis 12, **dadurch gekennzeichnet, dass** das Instrument (14) einen Schaft (50) aufweist, an dessen distalem Ende der Haltekörper (76) angeordnet oder ausgebildet ist.

14. Hüftgelenk-Endoprothesen-System nach Anspruch 14, **dadurch gekennzeichnet, dass**
a) das Instrument (14) eine in proximaler Richtung weisende Schlagfläche (58) aufweist, welche an einem proximalen Ende des Schafts (50) angeordnet oder ausgebildet ist
und/oder
b) der Schaft (50) die Längsachse (52) definiert.

15. Hüftgelenk-Endoprothesen-System nach einem der voranstehenden Ansprüche, **gekennzeichnet durch** eine Hüftgelenk-Endoprothese (20) umfassend mindestens eine Gelenkpfanne (18) und mindestens einen zu dieser korrespondierend ausgebildeten Gelenkeinsatz (16).

## Claims

1. Hip joint endoprosthesis system (10) with a medical instrumentarium for implanting a hip joint endoprosthesis (20) comprising at least one medical instrument (14) for inserting a joint insert (16) into a joint socket (18) of the hip joint endoprosthesis (20), wherein the instrument (14) defines a longitudinal axis (52) and comprises a holding element (84) for temporarily coupling to the joint insert (16) and a centering body (126) for centering the joint insert (16) relative to the holding element (84), wherein the holding element (84) has at least one retaining member (94) which has a retaining face (98), **characterized in that** the retaining face (98) is inclined with respect to the longitudinal axis (52), **in that** the retaining face (98) is configured in the form of an annular face (102) facing in the direction toward the longitudinal axis (52), and **in that** the retaining face (98) is configured in the form of an inner conical face (104) tapering in distal direction.

2. Hip joint endoprosthesis system in accordance with Claim 1, **characterized in that** the holding element (84) is formed rotationally symmetrically or substantially rotationally symmetrically with respect to the longitudinal axis (52).

3. Hip joint endoprosthesis system in accordance with Claim 1 or 2, **characterized in that** the holding element (84) comprises at least one joint insert stop (90) facing in distal direction for abutting the joint insert (16),
wherein in particular
a) the at least one joint insert stop (90) comprises an annular face (88) facing in distal direction
and/or
b) the centering body (126) is formed projecting in distal direction over the at least one joint insert stop (90).

4. Hip joint endoprosthesis system in accordance with any one of the preceding Claims, **characterized in that** the holding element has at least one undercut (106) with respect to a longitudinal axis (52) of the instrument (14).

5. Hip joint endoprosthesis system in accordance with any one of the preceding Claims, **characterized in that**
a) the holding element (84) is configured to be deformable at least in sections
and/or
b) the retaining member (94) is configured to be deformable at least in sections
and/or
c) the holding element (84) is integrally formed.

6. Hip joint endoprosthesis system in accordance with any one of the preceding Claims, **characterized in that**
a) the retaining face (98) is spaced apart from an outer face (134) of the centering body (126)
and/or
b) the retaining member (94) is able to be spread open from an initial position, in which it defines a cone shell wall (150) tapering in distal direction, into a release position, in which the retaining member (94) defines a cylinder shell wall (152) which is substantially concentric to the longitudinal axis (52).

7. Hip joint endoprosthesis system in accordance with any one of the preceding Claims, **characterized in that** the centering body (126) is of spherical or substantially spherical configuration or has at least one annular face (140) which forms a part of a spherical surface.

8. Hip joint endoprosthesis system in accordance with any one of the preceding Claims, **characterized in that** the instrument (14) comprises a holding body (76) on which the holding element (84) is arranged and projects facing in distal direction.

9. Hip joint endoprosthesis system in accordance with Claim 8, **characterized in that**
a) the holding element (84) is connected to the holding body (76) by way of at least two connecting members (82) extending substantially in radial direction
and/or
b) the holding body (76) is integrally formed.

10. Hip joint endoprosthesis system in accordance with Claim 8, **characterized in that** the centering body (126) is arranged or formed on the holding body (76) and protrudes facing in distal direction.

11. Hip joint endoprosthesis system in accordance with Claim 8 or 9, **characterized in that**
a) the centering body (126) and the holding body (76) are non-detachably connected to each other
and/or
b) the instrument (14) comprises a connecting device for connecting the centering body (126) and the holding body (76).

12. Hip joint endoprosthesis system in accordance with any one of the preceding Claims, **characterized in that** the centering body (128) has a support face (128) which faces in proximal direction and which the at least two connecting members (82) abut in the initial position.

13. Hip joint endoprosthesis system in accordance with any one of Claims 7 to 12, **characterized in that** the instrument (14) has a shank (50), on whose distal end the holding body (76) is arranged or formed.

14. Hip joint endoprosthesis system in accordance with Claim 14, **characterized in that**
a) the instrument (14) has a striking face (58) which faces in proximal direction and is arranged or formed on a proximal end of the shank (50)
and/or
b) the shank (50) defines the longitudinal axis (52).

15. Hip joint endoprosthesis system in accordance with any one of the preceding Claims, **characterized by** a hip joint endoprosthesis (20) comprising at least one joint socket (18) and at least one joint insert (16) formed correspondingly thereto.

## Revendications

1. Système à endoprothèse de l'articulation de la hanche (10) avec un instrumentarium médical (12) pour l'implantation d'une endoprothèse de l'articulation de la hanche (20) comprenant au moins un instrument médical (14) pour l'insertion d'un insert d'articulation (16) dans une cupule d'articulation (18) de l'endoprothèse de l'articulation de la hanche (20), où l'instrument (14) définit un axe longitudinal (52) et comprend un élément de maintien (84) pour le couplage temporaire avec l'insert d'articulation (16) et un corps de centrage (126) pour le centrage de l'insert d'articulation (16) par rapport à l'élément de maintien (84), où l'élément de maintien (84) présente au moins un organe de retenue (94) qui présente une surface de retenue (98), **caractérisé en ce que** la surface de retenue (98) est inclinée par rapport à l'axe longitudinal (52), **en ce que** la surface de retenue (98) est agencée sous forme d'une surface annulaire (102) tournée vers l'axe longitudinal (52) et **en ce que** la surface de retenue (98) est agencée sous forme d'une surface de cône intérieur (104) qui va en se rétrécissant dans la direction distale.

2. Système à endoprothèse de l'articulation de la hanche selon la revendication 1, **caractérisé en ce que** l'élément de maintien (84) est agencé de manière symétrique en rotation ou de manière sensiblement symétrique en rotation par rapport à l'axe longitudinal (52).

3. Système à endoprothèse de l'articulation de la hanche selon la revendication 1 ou 2, **caractérisé en ce que** l'élément de maintien (84) comprend au moins une butée d'insert d'articulation (90) tournée dans la direction distale pour l'appui sur l'insert d'articulation (16),
où en particulier
a) la au moins une butée d'insert d'articulation (90) comprend une surface annulaire (88) tournée dans la direction distale
et/ou
b) le corps de centrage (126) est agencé en saillie au-dessus de la au moins une butée d'insert d'articulation (90) dans la direction distale.

4. Système à endoprothèse de l'articulation de la hanche selon l'une des revendications précédentes, **caractérisé en ce que** l'élément de maintien présente au moins une contre-dépouille (106) par rapport à un axe longitudinal (52) de l'instrument (14).

5. Système à endoprothèse de l'articulation de la hanche selon l'une des revendications précédentes, **caractérisé en ce que**
a) l'élément de maintien (84) est agencé de manière déformable au moins par sections
et/ou
b) l'organe de retenue (94) est agencé de manière déformable au moins par sections
et/ou
c) l'élément de maintien (84) est agencé d'une pièce.

6. Système à endoprothèse de l'articulation de la hanche selon l'une des revendications précédentes, **caractérisé en ce que**
a) la surface de retenue (98) est distante d'une surface extérieure (134) du corps de centrage (126)
et/ou
b) l'organe de retenue (94) peut être déployé d'une position initiale, dans laquelle il définit une paroi d'enveloppe de cône (150) qui va en se rétrécissant dans la direction distale, dans une position de desserrage, dans laquelle l'organe de retenue (94) définit une paroi d'enveloppe de cylindre (152) sensiblement concentrique à l'axe longitudinal (52).

7. Système à endoprothèse de l'articulation de la hanche selon l'une des revendications précédentes, **caractérisé en ce que** le corps de centrage (126) est agencé de manière sphérique ou sensiblement sphérique ou présente au moins une surface annulaire (140), qui forme une partie d'une surface sphérique.

8. Système à endoprothèse de l'articulation de la hanche selon l'une des revendications précédentes, **caractérisé en ce que** l'instrument (14) comprend un corps de maintien (76) sur lequel est agencé l'élément de maintien (84) et fait saillie en étant tourné dans la direction distale.

9. Système à endoprothèse de l'articulation de la hanche selon la revendication 8, **caractérisé en ce que**
a) l'élément de maintien (84) est relié au corps de maintien (76) par l'intermédiaire d'au moins deux organes de liaison (82) qui s'étendent sensiblement dans la direction radiale
et/ou
b) le corps de maintien (76) est agencé d'une pièce.

10. Système à endoprothèse de l'articulation de la hanche selon la revendication 8, **caractérisé en ce que** le corps de centrage (126) est agencé ou formé sur le corps de maintien (76) et s'étend dans la direction distale.

11. Système à endoprothèse de l'articulation de la hanche selon la revendication 8 ou 9, **caractérisé en ce que**
a) le corps de centrage (126) et le corps de maintien (76) sont reliés l'un à l'autre de manière inamovible
et/ou
b) l'instrument (14) comprend un dispositif de liaison pour la liaison du corps de centrage (126) et du corps de maintien (76).

12. Système à endoprothèse de l'articulation de la hanche selon l'une des revendications précédentes, **caractérisé en ce que** le corps de centrage (128) présente une surface de soutien (128) tournée dans la direction proximale, sur laquelle s'appuient les au moins deux organes de liaison (82) dans la position initiale.

13. Système à endoprothèse de l'articulation de la hanche selon l'une des revendications 7 à 12, **caractérisé en ce que** l'instrument (14) présente une tige (50) à l'extrémité distale de laquelle le corps de maintien (76) est agencé ou formé.

14. Système à endoprothèse de l'articulation de la hanche selon la revendication 14, **caractérisé en ce que**
a) l'instrument (14) présente une surface de percussion (58) tournée dans la direction proximale, qui est agencée ou formée à une extrémité proximale de la tige (50)
et/ou
b) la tige (50) définit l'axe longitudinal (52).

15. Système à endoprothèse de l'articulation de la hanche selon l'une des revendications précédentes, **caractérisé par** une endoprothèse de l'articulation de la hanche (20) comprenant au moins une cupule d'articulation (18) et au moins un insert d'articulation (16) agencé d'une manière correspondant à celle-ci.
